## Europäisches Patentamt

## European Patent Office

## Office européen des brevets

(11) Veröffentlichungsnummer: **0 141 224**
**B1**

(12) **EUROPÄISCHE PATENTSCHRIFT**

(45) Veröffentlichungstag der Patentschrift:
12.08.87

(21) Anmeldenummer: 84111209.7

(22) Anmeldetag: 20.09.84

(51) Int. Cl.⁴: **C 12 N 11/08,** C 12 N 11/02,
C 12 N 11/06, C 12 N 9/16

(54) Verfahren zur enzymatischen Hydrolyse von Nukleinsäuren.

(30) Priorität: 27.09.83 DE 3334847

(43) Veröffentlichungstag der Anmeldung:
15.05.85 Patentblatt 85/20

(45) Bekanntmachung des Hinweises auf die Patenterteilung:
12.08.87 Patentblatt 87/33

(84) Benannte Vertragsstaaten:
AT BE CH DE FR GB IT LI NL SE

(56) Entgegenhaltungen:
EP-A-0 037 667
EP-A-0 052 365
DE-A-3 131 908

RUTTLOFF et al. "Industrielle Enzyme", 1. Auflage,
1979, DR. DIETRICH STEINKOPF VERLAG,
Darmstadt,"Nucleinspaltende Enzyme", 5.15.
Seiten 358-361
JOURNAL OF SOLID-PHASE BIOCHEMISTRY, Vol.
1, No. 1, März 1976, New York, S. NOGUCHI et al.
"Production of 5'-Mononucleotides using
immobilized 5'-Phosphodiesterase and 5'-AMP
Deaminase", Seiten 105-118

(73) Patentinhaber: HOECHST AKTIENGESELLSCHAFT,
Postfach 80 03 20, D-6230 Frankfurt am Main 80
(DE)

(72) Erfinder: Keller, Reinhold, Dr., Wiesenweg 5,
D-6233 Bad Soden am Taunus (DE)
Erfinder: Schlingmann, Merten, Dr., Schneidhainer
Strasse 32a, D-6240 Königstein/Taunus (DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

### Beschreibung

Die Immobilisierung von Enzymen ist schon mehrfach beschrieben worden: in der Britischen Patentschrift 1 369 462 ist beschrieben, daß man N-Acyl-D,L-aminosäuren mit Acylasen umsetzen kann, wobei selektiv die L-Aminosäure umgesetzt wird. Es wird erwähnt, daß die Acylase auch auf einem inerten Material wie Cellulose, Sand oder Aluminiumoxid adsorbiert sein kann und mit einem bifunktionalen Reagenz wie Glutardialdehyd vernetzt werden kann. In der Deutschen Offenlegungsschrift 2 732 301 wird die Immobilisierung von Proteinen beschrieben, wobei speziell auf das Enzym Penicillinacylase verwiesen wird. Nach der Deutschen Patentschrift 3 048 612 kann eine derart fixierte Penicillin-G-Acylase zur Racematspaltung von D,L-2-Amino-4-methylphosphinobuttersäure eingesetzt werden.

Die Europäische Patentanmeldung A1-43169 betrifft Enzympräparationen mit Inulinase-Aktivität. In einer bevorzugten Ausführungsform wird das Enzym durch Reaktion mit Glutardialdehyd an ein makroporöses Harz mit Amino- und/oder Hydroxygruppen gebunden. Als Beispiel wird ein Phenol-Formaldehyd-Harz mit Hydroxygruppen aufgeführt und erwähnt, daß auch ein Phenol-Formaldehyd-Harz mit Aminogruppen Verwendung finden könne. Weiterhin wird erwähnt, daß zur Bindung des Enzyms an einen Träger als bifunktionale Reagenzien neben Dialdehyden auch Dicarbonsäuren, deren Anhydride oder Diisocyanate in Betracht kommen.

In der EP-A1-43169 ist der Phenol-Formaldehyd-Harz-Träger mit Aminogruppen durch das Warenzeichen $^R$DUOLITE A-7 charakterisiert. Nach der Europäischen Patentanmeldung A2-37667 handelt es sich hierbei um ein makroporöses Anion-Austauscherharz auf Phenol-Formaldehydbasis mit einem Teilchendurchmesser von 250 - 840 µm, 31 m²/g spezifischer Oberfläche, 0,52 cm³/g Gesamtvolumen der Makroporen. die einen Porendurchmesser von 10 - 200 nm aufweisen. und 7,5 meq/g Anion-Austauscherkapazität aufgrund von primären und sekundären Aminogruppen. Dieses Produkt wird von der Firma Diamond Shamrock vertrieben. Als allgemein geeignet werden granulat- oder perlförmige Anion-Austauscherharze auf Phenol-Formaldehydbasis beschrieben, deren Größe vorzugsweise im Bereich von 250µm bis 1,4 mm liegt.

Aus der Europäischen Patentanmeldung A2-75262 ist es bekannt, 5'-Ribonucleotide dadurch herzustellen, daß man die Ribonucleinsäure (RNA) in Rohnucleinsäurelösungen mit einer an einen polymeren Träger immobilisierten 5'-Phosphodiesterase selektiv hydrolysiert, wobei also die Desoxyribonucleinsäure (DNA) unverändert bleibt. Bevorzugt wird ein makroporöser Träger mit Kanälen mit einem Durchmesser bis zu 100 nm und einem Porenvolumen von etwa 2 bis 3 ml/g. Die Immobilisierung von 5'-Phos-phodiesterase war schon früher beschrieben worden, wobei jedoch nichts über die Selektivität der Hydrolyse ausgesagt ist (S. Noguchi et al., Journal of Solid-Phase Biochemistry 1, 105 (1976)).

Es wurde nun gefunden, daß die Fixierung von 5'-Phos-phodiesterase an einen speziellen Träger ein sehr aktives Enzym liefert, das generell Nucleinsäuren zu Nucleotiden spaltet, also auch hochmolekulare Desoxyribonucleinsäure. Die Erfindung betrifft somit ein Verfahren zur enzymatischen Hydrolyse von Nukleinsäuren, das dadurch gekennzeichnet ist, daß hochmolekulare Nukleinsäure mit 5'-Phosphodiesterase, die an ein mikroporöses Anionenaustauscherharz auf Basis Phenol/Formaldehyd mit im wesentlichen sekundären Aminogruppen mit Hilfe eines Diisocyanats immobilisiert ist, behandelt wird.

Erfindungsgemäß wird die 5'-Phosphodiesterase an Anion-Austauscherharze auf Phenol-Formaldehyd-Basis mit im wesentlichen sekundären Aminogruppen mit Hilfe eines Diisooyanats gebunden. Bevorzugt wird ein entsprechendes Harz mit einem Teilchendurchmesser von 250-840 µm und einem Porendurchmesser von 10-200 nm eingesetzt. Von besonderem Vorteil ist, daß zur Bindung des Enzyms an den Träger kein Katalysator erforderlich ist, wie es beispielsweise bei den in der Europäischen Patentanmeldung EP-A1-52365 genannten Methoden der Fall ist.

Als Diisocyanate kommen aromatische, cycloaliphatische und insbesondere aliphatische Diisocyanate in Betracht, wie sie beispielsweise in der EP-A1-52365 beschrieben sind. Bevorzugt sind handelsübliche aliphatische Diisocyanate, wie Alkylendiisocyanat mit bis zu 10 Kohlenstoffatomen in der Alkylenkette, z. B. das 2,2,4- und 2,4,4-Trimethyl-hexa-methylen-1,6-diisocyanat und insbesondere das Hexamethylen-1,6-diiso-cyanat.

Zur Kopplung des Enzyms an den Ionenaustauscher wird dieser zunächst in die freie Basenform überführt, zweckmäßig durch Suspendieren in einer starken wäßrigen Base wie Natronlauge. Nach Neutralwaschen und Trocknen wird das wasserfreie Harz in einem aprotischen Lösemittel für das Diisocyanat aufgenommen und das Diisocyanat in Substanz oder in Lösung zugesetzt und gut vermischt. Das Umsetzungsprodukt kann nach Abtrennen von der Reaktionslösung und Waschen mit dem Lösemittel unmittelbar zu der Umsetzung mit dem Enzym verwendet werden. Es kann aber auch isoliert und getrocknet werden und ist unter Feuchtigkeitsausschluß längere Zeit haltbar.

Das so vorbehandelte Harz kann unmittelbar mit einer wäßrigen Lösung des Enzyms ungesetzt werden, wobei sich die Reaktionsbedingungen nach den Eigenschaften des Enzyms richten. Im allgemeinen wird das vorbehandelte Harz mit der wäßrigen Enzymlösung ausreichend lange bei Raumtemperatur verrührt, das Produkt abfiltriert und gewaschen, beispielsweise mit gesättigter oder physiologischer Kochsalzlösung und anschließend mit Wasser. Das gewaschene Enzymimmobilisat kann unmittelbar eingesetzt werden.

Als Ausgangsmaterial eignen sich DNA-Lösungen, wie sie nach dem Verfahren der EP A2-75262 erhalten werden. Ein weiteres geeignetes Ausgangsmaterial kann durch Auftrennung von Rohnucleinsäurelösungen in einem Membrantrennverfahren erhalten werden, wobei die DNA als Retentat zurückbleibt und die RNA permeiert. Bevorzugt ist die Ultrafiltration an Hohlfaser-Membranen (Deutsche Offenlegungsschrift 33 08 932).

In den folgenden Beispielen werden bevorzugte Ausgestaltungen der Erfindung näher erläutert. Prozentangaben beziehen sich auf das Gewicht, wenn nichts anderes angegeben ist.

### Beispiel 1

Das handelsübliche makroporöse Anionen-Austauscherharz auf Basis Phenol-Formaldehyd mit im wesentlichen sekundären Aminogruppen DUOLITE A-7 wird zur Überführung in die freie Basenform in 2N Natronlauge aufgenommen, gerührt und mit entionisiertem Wasser neutral gewaschen. Nach Trocknen im Vakuumschrank (40°C, 50mbar, 24 Stunden) werden 100 g des wasserfreien Harzes in 200 ml wasserfreiem Dichlormethan aufgenommen, mit 10 g Hexamethylen-1,6-diisocyanat versetzt und 2 Stunden unter Feuchtigkeitsausschluß bei Raumtemperatur gerührt. Anschließend filtriert man ab und wäscht das Harz mit Dichlormethan gründlich nach. Das Harz wird unter Feuchtigkeitsausschluß von Dichlormethan befreit und unter Feuchtigkeitsausschluß bis zum Einsatz aufbewahrt.

### Beispiel 2

5 g 5'-Phosphodiesterase (Nuclease Rp der Fa. Amano) werden in 200 ml 0,1 N wäßrigem Acetatpuffer (pH 5,5) gelöst. Zur Lösung gibt man unter Rühren 100 g des nach Beispiel 1 behandelten Harzes und rührt weitere 2 Stunden bei Raumtemperatur. Das Produkt wird abfiltriert und zunächst mit 0,1 N Acetat-Puffer (pH 5,5), hierauf mit gesättigter Kochsalzlösung und erneut mit dem Acetatpuffer gewaschen. Das abfiltrierte Produkt wird in eine Säule gefüllt.

Zur Messung der Aktivität wird bei 55°C durch das immobilisierte Enzym eine Substratlösung, bestehend aus 4 % RNA und 0,1 mm Zinksulfat (pH 5,0), mit einer Durchflußgeschwindigkeit (SV) von 20 bis 30 $h^{-1}$ gepumpt. Die Menge der entstehenden Mononucleotide wird photometrisch oder durch Hochdruckflüssigkeitschromatographie ermittelt.

### Beispiel 3

200 g DNA werden in 10 l Wasser aufgenommen und durch Einstellen des pH-Werts auf 5,0 in Lösung gebracht. Die lösung wird mit 500 mg Zinksulfat-Heptahydrat versetzt und bei 55°C über eine Säule, gefüllt mit 500 ml einer nach Beispiel 2 immobilisierten 5'-Phosphodiesterase, gegeben. Die Durchflußgeschwindigkeit wird so eingestellt, daß gerade ein 100 %iger Abbau der DNA zu den 5'-Desoxynucleotiden erfolgt (5 l/Std.).

Die Isolierung und Trennung der 4 verschiedenen 5'-Des-oxynucleotide kann in bekannter Weise durch Ionenaustauschchromatographie oder Adsorption erfolgen.

Als Ausgangsmaterial eignet sich eine DNA nach Beispiel 2 der EP A2-75262 oder nach Beispiel 1 oder 2 der Deutschen Offenlegungsschrift 33 08 932.

### Patentansprüche

1. Verfahren zur enzymatischen Hydrolyse von Nukleinsäuren, dadurch gekennzeichnet, daß hochmolekulare Nukleinsäuren mit 5'-Phosphodiesterase, die an ein makroporöses Anionenaustauscherharz auf Basis Phenol/Formaldehyd mit im wesentlichen sekundären Aminogruppen mit Hilfe eines Diisocyanats immobilisiert ist, behandelt wird.

2. Verfahren nach Anspruch 1, dadurch gekennzeichnet, daß ein Trägerharz mit einem Porendurchmesser von 10 bis 200 nm und einem Teilchendurchmesser von 250 bis 840 µm eingesetzt wird.

3. Verfahren nach Anspruch 1 oder 2, dadurch gekennzeichnet, daß als Diisocyanat ein Alkylendiisocyanat mit bis zu 10 Kohlenstoffatomen in der Alkylenkette eingesetzt wird.

### Claims

1. A process for enzymatic hydrolysis of nucleic acids, wherein high molecular nucleic acids are treated with 5'-phosphodiesterase immobilized by means of a diisocyanate on a macroporous anion exchange resin based on phenol/formaldehyde carrying essentially secondary amino groups.

2. The process as claimed in claim 1, wherein a carrier resin having a pore diameter of 10 to 200 nanometers and a particle diameter of 250 to 840 µm is used.

3. The process as claimed in claims 1 or 2, wherein the diisocyanate used is an alkylene diisocyanate having up to 10 carbon atoms in the alkylene chain.

## Revendications

1. Procédé pour l'hydrolyse enzymatique d'acides nucléiques, caractérisé en ce que l'on traite des acides nucléiques a haut poids moléculaire avec une 5'-phospho-diestérase qui est immobilisée, à l'aide d'un diisocyanate, sur une résine macroporeuse échangeuse d'anions, à base de phénol et de formaldéhyde, comportant des groupes amino essentiellement secondaires.

2. Procédé selon la revendication 1, caractérisé en ce que l'on utilise une résine de support ayant un diamètre de pores de 10 à 200 nm et un diamètre de particules de 250 à 840 μm.

3. Procédé selon la revendication 1 ou 2, caractérisé en ce que l'on utilise en tant que diisocyanate un diisocyanate d'alkylène ayant jusqu'à 10 atomes de carbone dans la chaîne alkylène.